# EUROPÄISCHE PATENTSCHRIFT

(11) **EP 1 718 963 B1**
(45) Veröffentlichungstag und Bekanntmachung des Hinweises auf die Patenterteilung: **19.08.2009**
(21) Anmeldenummer: 05707181.3
(22) Anmeldetag: 31.01.2005
(51) Int. Cl.: G01N 33/00

(54) **VERFAHREN UND VORRICHTUNG ZUR CHARAKTERISIERUNG VON OSI-MATERIAL**
METHOD AND DEVICE FOR CHARACTERIZING OSI MATERIALS
PROCEDE ET DISPOSITIF POUR CARACTERISER UN MATERIAU OSI

(30) Priorität: 26.02.2004 DE 102004009870
(43) Veröffentlichungstag der Anmeldung: 08.11.2006
(73) Patentinhaber: Fraunhofer-Gesellschaft zur Förderung der angewandten Forschung e.V., 80686 München (DE)
(72) Erfinder: WANNER, Thomas, 86529 Schwabenhausen (DE); RODLER, Norbert, 85406 Zolling (DE); RIEBLINGER, Klaus, 85399 Hallbergmoos (DE); HUBENSTEINER, Thomas, 85354 Freising (DE)
(86) Internationale Anmeldenummer: PCT/EP2005/001099
(87) Internationale Veröffentlichungsnummer: WO 2005/085836

(56) Entgegenhaltungen:
- DE-C1- 19 528 400
- US-A- 4 947 339
- US-A- 5 358 876
- US-A1- 2003 082 321
- US-B1- 6 455 620
- RIEBLINGER K: "Qualitätsvorteile. Sauerstoffzehrende Verpackungen gegen unerwünschte Oxidationsreaktionen" LEBENSMITTELTECHNIK, Bd. 35, Nr. 5, 2003, Seiten 66-67, XP009050469 ISSN: 0047-4290

## Beschreibung

Die Erfindung betrifft ein Verfahren zur Charakterisierung von OSI-Material nach dem Oberbegriff des Hauptanspruchs und eine entsprechende Vorrichtung nach dem Oberbegriff des Nebenanspruchs.

Es sind verschiedene analytische Geräte bzw. Verfahren zur Charakterisierung von unter dem Begriff OSI-Materialien fallenden 02-Scavenger bekannt. In der Gas-Chromatographie wird beispielsweise eine bestimmte Menge eines O₂-Scavengers einem Gasgemisch ausgesetzt, das Sauerstoff enthält. Nach einer gewissen Zeit, in der der O₂-Scavenger Sauerstoff aus dem Gasgemisch abbaut, wird eine bestimmte Menge des vorhandenen Gasgemischs einer Messeinrichtung zugeführt, in der das zu analysierende Gas über eine Trennsäule vom Gasgemisch separiert und über einen Detektor, z.B. einem Helium Pulsed-Discharged-Detektor quantifiziert wird.

Weiterhin sind beispielsweise elektrochemische Verfahren, wie z.B. O₂-sensitive Elektroden, die über eine elektrochemische Reaktion die Sauerstoffänderung in einem Kompartiment detektieren. Auch gibt es Druckmessverfahren, bei denen über einen Drucksensor die Druckänderung in einem starren Kompartiment detektiert wird. Dabei ist die Druckänderung direkt proportional zur durch den O₂-Scavenger hervorgerufenen Sauerstoffänderung. Außerdem ist die Fluoreszenz-Messtechnik anwendbar, bei der die Fluoreszenzlöschung eines aktiv angeregten O₂-sensitiven Farbstoffs detektiert wird. Die Fluoreszenzlöschung ist indirekt proportional zur Sauerstoffkonzentration.

Aus der US 6,455,620 B1 sind O₂-Scavenger enthaltende Polymere sowie ein Verfahren zur Bestimmung der Sauerstoffzehrenden Wirkung der Polymerfilme mittels eines "closed-circuit" Respirometers bekannt.

Zur Charakterisierung von O₂-Indikatoren, die gleichfalls ein OSI-Material darstellen, gibt es derzeit keine spezielle Messtechnik.

O₂-Scavenger sind Stoffe, die Sauerstoff absorbieren und/oder adsorbieren. Die derzeit am Markt etablierten Systeme lassen sich primär nach dem O₂-Scavenger-Substrat und nach ihrem Initialisierungsmechanismus klassifizieren. Bei der Klassifizierung durch das O₂-Scavenger-Substrat wird unterschieden nach anorganischen O₂-Scavenger, z.B. eisenbasierte oder sulfitbasierte Systeme, nach niedermolekularen organischen O₂-Scavenger, z.B. ascorbatbasierte Systeme und nach hochmolekularen organischen O₂-Scavengern (z.B. polyolefinbasierte oder polyamidbasierte Systeme). Die O₂-Scavenger werden entweder UV-initialisiert oder Feuchte-initialisiert. Das bedeutet, dass die O₂-Scavengerfunktion erst nach einer Exposition mit UV-Licht bzw. Feuchte vorhanden ist.

Indikatorsysteme im Allgemeinen lassen sich in Zeit-Temperatur-Indikator- (Time-Temperature-Indicator (TTI)), "Gas/Leakage-Indicator"- und "Freshness-Indicator"-Systeme einteilen. Zeit-Temperatur-Indikatoren integrieren die Zeit-Temperaturgeschichte eines Produktes und machen somit eine direkte Aussage über dessen Lagerbedingungen. Die Indikatorwirkung wird durch eine chemische Reaktion oder durch gegenläufige Diffusion zweier Farbstoffe bewirkt. "Gas/Leakage-Indikatoren" detektieren die Gaskonzentration, z.B. O₂, CO₂ oder H₂O im Verpackungsraum eines Produktes. Sie machen somit eine indirekte Aussage über die Qualität des Produktes. Die Indikatorwirkung wird durch eine chemische Reaktion mit dem entsprechenden Gas als Reaktand hervorgerufen. "Freshness Indikatoren" detektieren die Stoffwechselprodukte von Mikroorganismen und machen somit eine direkte Aussage. über die Qualität des Produktes. Die Indikatorwirkung wird durch eine chemische Reaktion der Stoffwechselprodukte hervorgerufen. Alle genannten Indikatorsysteme geben ihre Indikatorwirkung durch einen sichtbaren Farbumschlag wieder.

OSI-Materiälien, nämlich O₂-Scavenger, O₂-Indikatoren oder O₂-Scavenger/O₂-Indikatorsysteme finden in der Lebensmittelindustrie, der pharmazeutischen Industrie, der Elektronikindustrie, der chemischen Industrie und anderen Anwendung. Um diese OSI-Materialien in ihrer Menge, ihrer Wirkung und anderen Parametern an die Anforderungen der entsprechenden Zielsetzungen anpassen zu können, ist es notwendig, das jeweilige OSI-Material zu charakterisieren, wobei die Grundlage der Charakterisierung die Sauerstoffkonzentration in Bezug auf eine Zeitkomponente ist.

Der Erfindung liegt daher die Aufgabe zugrunde ein Verfahren und eine Vorrichtung zur Charakterisierung von OSI-Materialien zu schaffen, mit denen in relativ einfacher Weise und mit großer Empfindlichkeit OSI-Materialien, einschließlich O₂-Indikatoren charakterisiert werden könnten.

Die Aufgabe wird erfindungsgemäß durch die kennzeichnenden Merkmale des Hauptanspruchs sowie des Nebenanspruchs in Verbindung mit deren Oberbegriffen gelöst.

Dadurch, dass das OSI-Material in einer Messzelle ausgenommen ist, die in einem geschlossenen Reaktionskreislauf einem Sauerstoff enthaltenden Gasgemisch ausgesetzt wird und ein definierter Volumenteil in einem Gas enthaltenden geschlossenen Messkreislauf zur Messung der Sauerstoffkonzentration überführt wird, kann die Sauerstoffkonzentration in empfindlicher Weise gemessen werden. Durch den geschlossenen Reaktionskreislauf können sehr geringe Konzentrationen wirksam erfasst werden. Die entsprechende Vorrichtung ist einfach aufgebaut und gut zu handhaben.

Durch die in den Unteransprüchen angegebenen Maßnahmen sind vorteilhafte Weiterbildungen und Verbesserungen möglich.

Besonders vorteilhaft ist, dass dem geschlossenen Reaktionskreislauf über eine externe Befeuchtungseinheit Feuchte zugeführt werden kann, durch die der O₂-Scavenger initialisiert werden kann. Dadurch kann eine Charakterisierung des O₂-Scavengers bei jeder beliebigen relativen Feuchte durchgeführt werden.

Durch Vorsehen einer für vorgegebene Wellenlängenbereiche, z.B. UV/VIS oder sichtbares Licht durchlässige Messzelle kann der in der Messzelle enthaltende O₂-Scavenger mit UV-Strahlung beaufschlagt werden und dadurch initialisiert werden.

Bei Vorsehen eines O₂-Indikators und/oder eines O₂-Scavenger/O₂-Indikatorsystems kann dessen Farbe bzw. Farbänderung gemessen werden, die zur Charakterisierung des O₂-Indikators bzw. des Systems verwendet werden.

Dadurch, dass die Bauteile des Reaktionskreislaufs und besonders des Messkreislaufs vorteilhafterweise gekapselt sind, sind sehr geringe Leckraten zu verzeichnet, wodurch sehr geringe Konzentrationen, z.B. zwischen 20, und 0% Sauerstoff gemessen werden können.

Durch Einbringen von kompletten Verpackungen und Getränkeflaschen, die OSI-Materialien enthalten, können diese komplett charakterisiert werden. Der Messkreislauf ist gleichfalls ein geschlossener Kreislauf, und Reaktionskreislauf und Messkreislauf sind über eine in den jeweiligen Kreislauf schaltbare Probeschleife miteinander verbunden. Dadurch kann in einfacher Weise ein definierter Volumenanteil vom Reaktionskreislauf in den Messkreislauf überführt werden.

Ein Ausführungsbeispiel der Erfindung ist in der Zeichnung dargestellt und wird in der nachfolgenden Beschreibung näher erläutert. Es zeigen:
- Fig. 1: die schematische Darstellung der erfindungsgemäßen Vorrichtung in einem ersten Verfahrenszustand,
- Fig. 2: die Vorrichtung entsprechend Fig. 1 in einem zweiten Verfahrenszustand,
- Fig. 3: die Vorrichtung entsprechend Fig. 1 und Fig. 2 in einem dritten Verfahrenszustand, und
- Fig. 4: eine Kennlinie der Sauerstoffaufnahme eines O₂-Scavengers in Abhängigkeit von der Zeit, die zur Charakterisierung des Materials dient.

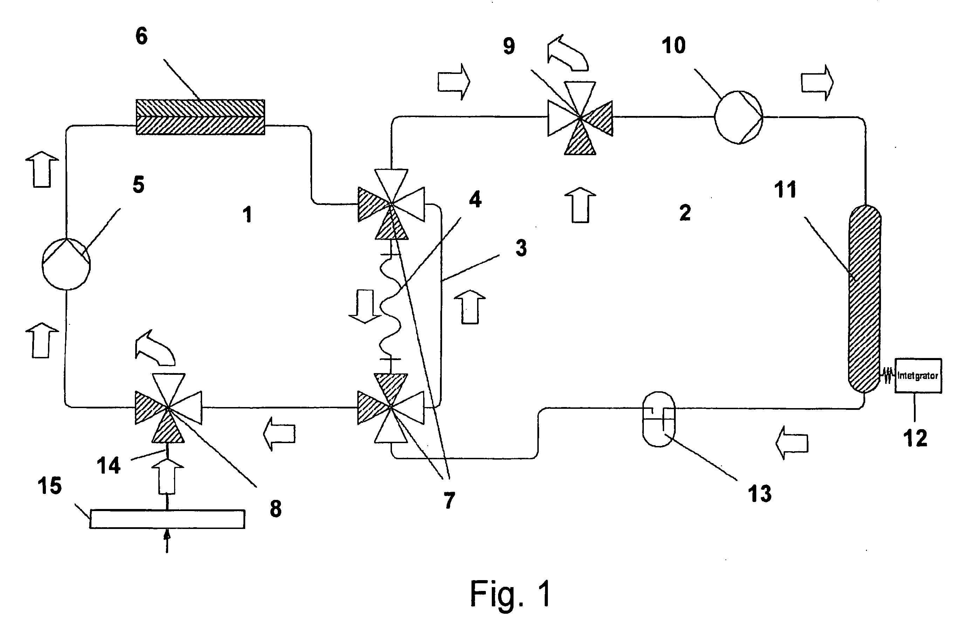

Die erfindungsgemäße Vorrichtung zur Charakterisierung von OSI-Materialien ist in den Fign. 1 bis 3 schematisch dargestellt, wobei die unterschiedlichen Figuren unterschiedliche Verfahrensfließzustände repräsentieren. Die Vorrichtung besteht aus einem Reaktionskreis 1 und einem Messkreis 2. Ein erster Umschaltzweig 3 und ein zweiter Umschaltzweig, der als Probeschleife 4 bezeichnet wird, ist jeweils zwischen dem Reaktionskreis 1 und dem Messkreis 2 umschaltbar.

Der Reaktionskreislauf 1 bildet einen geschlossenen Reaktionskreislauf und setzt sich aus einer Pumpe 5, einer transparenten bzw. für ausgewählte Wellenlängenbereiche durchlässige Messzelle 6 der Probeschleife 4 bzw. dem Umschaltkreis 3, einem 6-Wegeventil 7, das als simuliertes 6-Wegeventil dargestellt ist (Ersatzschaltbild aus zwei 4-Wegeventilen und Umschaltzweig) und zwischen der Probeschleife 4 und dem Umschaltzweig 3 umschaltet, und einem 4-Wegeventil zusammen.

Der Messkreis 2 ist im dargestellten Ausführungsbeispiel gleichfalls als geschlossener Kreislauf ausgebildet, in anderen Ausführungsformen, die nicht Teil der Erfindung sind, ist es denkbar, dass der Messkreis ein offener Kreis oder Zweig ist. Der Messkreis weist ein 4-Wegeventil 9 zur Abfuhr, Zufuhr und zum Durchschalten eines Gasstroms, eine Pumpe 10, eine sauerstoffsensitive Sensoranordnung 11, die beispielsweise einen Mocon-Sensor oder einen Coulox-Sensor aufweisen kann, die auf einem elektrochemischen Prinzip beruhen (der Mocon-Sensor baut beim Kontakt mit Sauerstoff durch elektrochemische Vorgänge eine Potentialdifferenz auf, wobei diese physikalische Größe mit der Sauerstoffkonzentration korreliert wird), eine Auswerteeinheit 12, die mit der Sensoranordnung 11 in Verbindung steht und z.B. einen Integrator enthalten kann, und eine Befeuchtungseinheit 13 auf. Wie schon oben erwähnt, ist das 6-Wegeventil 7, das zwischen Probeschleife 4 und Umschaltzweig 3 umschaltbar ist, auch Bestandteil des Messkreises 2.

Das 4-Wegeventil 8 des Reaktionskreises 1 dient gleichfalls zur Zufuhr, Abfuhr und Durchschalten eines Gasstroms, wobei eine zufuhrleitung14 mit einer externen Befeuchtungseinheit 15 verbünden ist.

Die dargestellte Vorrichtung bzw. das analytische Gerät ermöglicht eine Charakterisierung von reinen OSI-Materialien (z.B. Substanzen in Pulverform), aber auch von OSI-Materialien, die in unterschiedliche Matrizes, z.B. Polymere, eingearbeitet sind. Die Ausbildung der Matrizes kann in Form der eigentlichen Verpackungen (z.B. Folien in Mono- oder Multilayer-Aufbauten) und Getränkeflaschen realisiert sein.

Das zu charakterisierende OSI-Material, das in dem im Folgenden beschriebenen Beispiel ein O₂-Scavenger sein soll, wird in die Messzelle 6 eingebracht. Anschließend wird der gesamte Reaktionskreis mit einem O₂/N₂-Gasstrom, der beispielsweise 20% Sauerstoff und 80% Stickstoff enthalten kann und der über das 4-Wegeventil 8 zugeführt wird, gespült. Dies bedeutet, dass der zugeführte definierte Gasstrom, von der Pumpe 5 gefördert, durch die Messzelle 6 die Probeschleife 4, die über das 6-Wegeventil in den Reaktionskreislauf geschaltet ist, hindurchströmt und über das 4-Wegeventil 8 abgeführt wird, bis der gesamte Reaktionskreislauf neben dem definierten Gasstrom kein Fremdgas mehr enthält. Dies ist in Fig. 1 dargestellt, wobei die "hellen" Pfeile das abzuführende Fremdgas darstellen. Gegebenenfalls kann bei ausgeschalteter Pumpe der definierte Gasstrom durch den Überdruck der Gasflasche zugeführt werden, wobei die Durchflussgeschwindigkeit durch einen Durchflussmesser eingestellt wird.

Wenn es sich bei dem in der Messzelle 6 aufgenommenen O₂-Scavenger um einen Feuchte-initialisierenden O₂-Scavenger handelt, so wird der über das 4-Wegeventil 8 zugeführte definierte Gasstrom vorher durch die externe Befeuchtungseinheit 15 geleitet und mit der nötigen relativen Feuchte beaufschlagt, um den in der Messzelle 6 enthaltenen O₂-Scavenger zu initialisieren.

Im Fall eines UV-initialisierenden O₂-Scavengers muss die Messzelle 6 zumindest für die-UV-Strahlung durchlässig sein, und ihr ist eine nicht dargestellte UV-Strahlungsquelle zugeordnet, die den Scavenger bestrahlt und somit initialisiert.

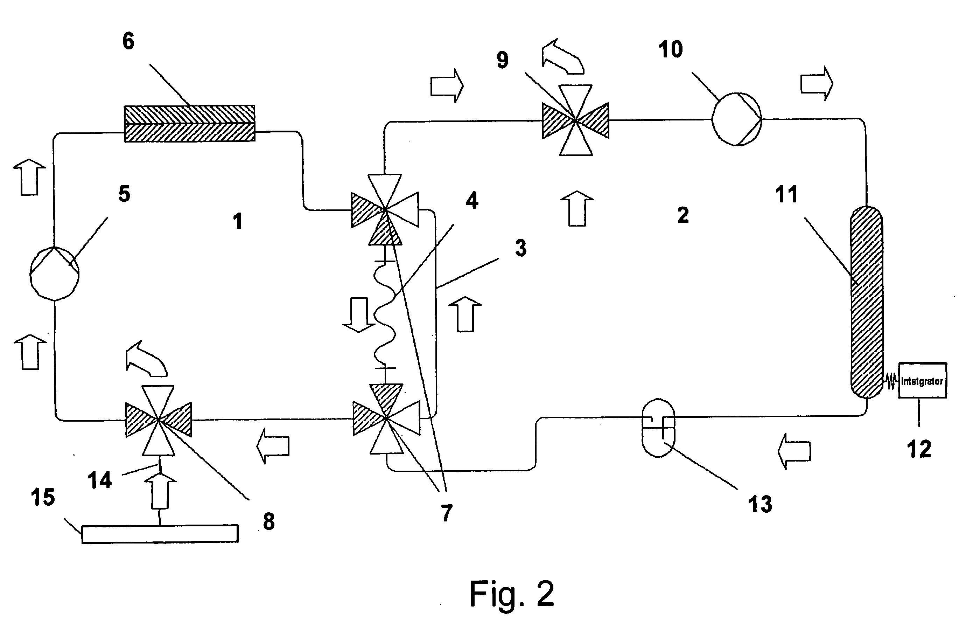

Nachdem der Reaktionskreis 1 ausreichend gespült wurde, wird das 4-Wegeventil 8 umgeschaltet und die zu diesem Zeitpunkt eingebrachte Gasmenge wird mit Hilfe der Pumpe 5 im Reaktionskreislauf umgewälzt. Dies ist in Fig. 2 zu erkennen.

Dem Messkreis 2 wird über das 4-Wegeventil 9 ein Stickstoff-Gasstrom, vorzugsweise 100% Stickstoff, zugeführt und durch die Pumpe 10 weitergeleitet, wobei das 6-Wegeventil 7 den Umschaltzweig 3 in den Messkreis 2 schaltet. Fremdgas wird aus dem Messkreis 2 über das 4-Wegeventil 9 herausgeführt, wie durch die weißen Pfeile angedeutet wird. Wenn das Fremdgas vollständig abgeführt ist, wird das 4-Wegeventil 9 umgeschaltet, derart, dass die eingebrachte Gasmenge mit Hilfe der Pumpe 10 im Messkreislauf umgewälzt wird. Dies ist in Fig. 2 dargestellt. Die Befeuchtungseinheit 13 hat ausschließlich die Aufgabe, den sauerstoffsensitiven Sensor der Sensoranordnung 11 zu befeuchten.

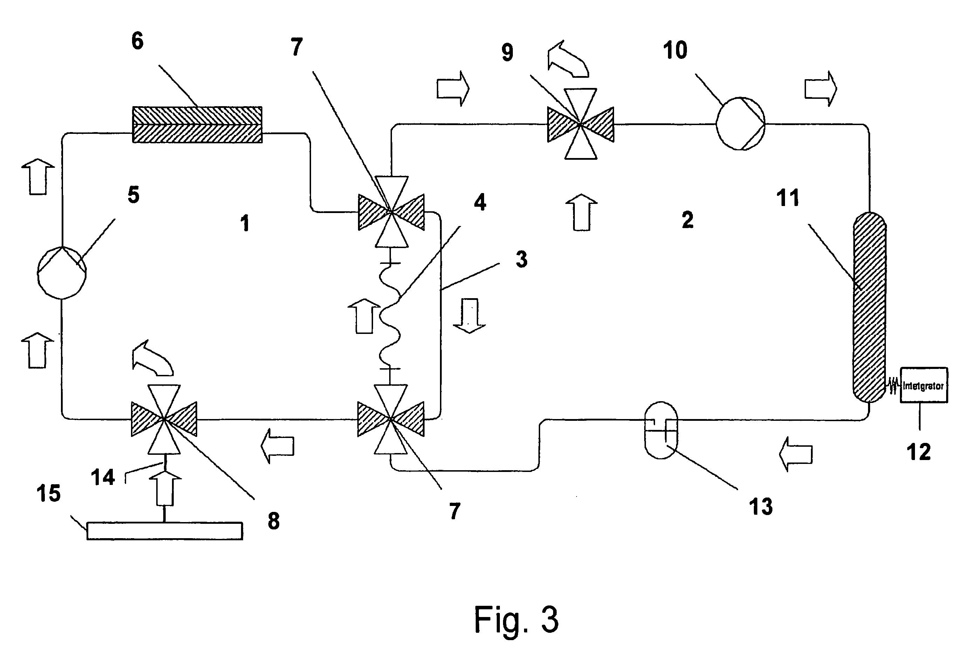

Aufgrund der O₂-Sorption des in der Messzelle 6 aufgenommenen O₂-Scavenger-Materials ändert sich im Reaktionskreis der Sauerstoffgehalt_des umgewälzten Gases. Zur Bestimmung der Sauerstoff-Konzentration wird in bestimmten Zeitabständen, z.B. in Intervallen von 24 Stunden, die Probeschleife 4 über das 6-Wegeventil 7 vom Reaktionskreis 1 in den Messkreis geschaltet. Dies ist in Fig. 3 dargestellt. Durch das Umschalten des 6-Wegeventils wird ein definierter Volumenteil vom Reaktionskreislauf 1 in den Messkreislauf 2 überführt und der in dem definierten Volumenteil enthaltene Sauerstoff wird mit Hilfe der Sensoranordnung 11 detektiert. Die Auswerteeinheit 12, die einen Integrator enthält, bestimmt dann die Sauerstoff-Konzentration des Reaktionskreises unter Heranziehung der im Reaktionskreis 1 und im Messkreis 2 enthaltenen Gasmengen. Der aliquote Volumenteil, der vom Reaktionskreis 1 in den Messkreis 2 überführt wird, führt bei dem Sensor 11 zu einem Signal (Flächensignal). Dies entspricht einer gewissen Sauerstoffkonzentration im Reaktionskreis. Dies bedeutet, dass jedem Sensorsignal eine bestimmte Sauerstoffkonzentration im Kreislauf zugeordnet ist.

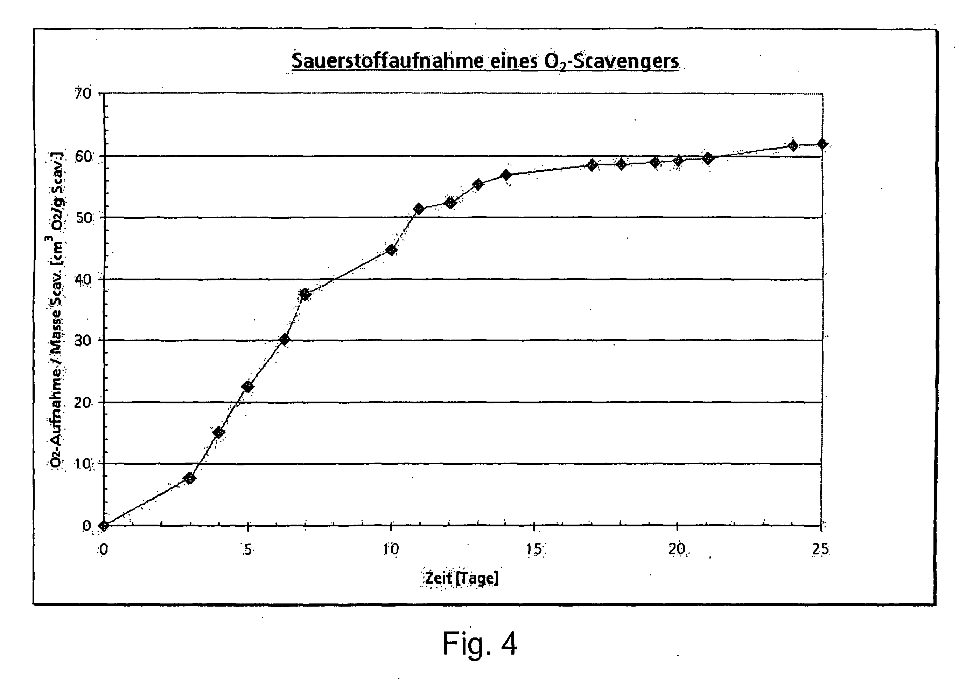

Die Charakterisierung des Materials des in diesem Ausführungsbeispiel verwendeten O₂-Scavengers basiert auf der Ermittlung der Kapazität und der Kinetik. Das bedeutet, dass die Auswerteeinheit beispielsweise unter Heranziehung der Menge des in der Messzelle 6 aufgenommenen O₂-Scavengers dessen Sauerstoffaufnahme über die Zeit bestimmt. Eine solche Kennlinie ist in Fig. 4 dargestellt, bei der die Ordinate den Sauerstoffabnahme bezogen auf die Masse des Scavengers zeigt, und die Abszisse die Zeit in Tagen darstellt. Als Kapazität wird üblicherweise der am Ende der Messreihe vorhandene bezogene Sauerstoff-Abbauwert bezeichnet und die Kinetik wird vereinfacht durch die Steigung der Kennlinie ausgedrückt.

Für die Charakterisierung eines O₂-Indikators wird in entsprechender Weise vorgegangen. Der O₂-Indikator wird in die Messzelle 6 eingebracht und jeweils der gesamte Reaktionskreislauf über das 4-Wegeventil,8. mit einem definierten O₂/N₂-Gasstrom gespült. Je nach Art der Initialisierung wird entweder der Indikator über den mit Feuchte beaufschlagten Gasstrom oder über eine UV-Strahlung über die UV durchlässige Messzelle 6 initialisiert. Im Anschluss daran wird die eingebrachte Gasmenge mit Hilfe der Pumpe 5 im Realtionskreislauf 1 umgewälzt.

Der Messkreislauf 2 wird, wie oben beschrieben, über das 4-Wegeventil 9 mit einem N₂-Gasstrom gespült. Im Anschluss wird die eingebrachte Gasmenge mit Hilfe der Pumpe 10 im Messkreislauf umgewälzt (siehe Fig. 2). Zur Bestimmung der Sauerstoffkonzentration im Reaktionskreislauf 1 wird in bestimmten Zeitintervallen, z.B. 24 Stunden, die Probeschleife 4 über das 6-Wegeventil 7 vom Reaktionskreis 1 in den Messkreis 2 geschaltet (Fig. 3). Durch diese Überführung eines aliquoten bzw. definierten Volumenteils vom Reaktionskreis 1 in den Messkreis 2 wird die jeweilige, zu dem Zeitpunkt vorhandene O₂-Konzentration des Reaktionskreislaufs mit Hilfe des sauerstoffsensitiven Sensors 11 und der Auswerteeinheit 12 detektier und bestimmt .

Zu den Zeitpunkten, in denen der Sauerstoffgehalt gemessen wird, wird die Farbe bzw. die Farbänderung des O₂-Indikatormaterials in der Messzelle festgestellt bzw. gemessen. Beispielsweise kann der Messzelle ein Farbmessgerät oder ein Fotometer oder dergleichen zugeordnet werden, das auf die transparente Messzelle aufgesetzt wird. Eine weitere Möglichkeit ist ein Farbabgleich mit einer Farbskala.

Für eine Charakterisierung des O₂-Indikators wird die Farbänderung des Indikators über die Sauerstoffkonzentrations-Zeitspanne bzw. Sauerstoff(Konzentrations)-Schwellenwert verwendet. Dazu ermittelt die Auswerteeinheit das Integral der O₂-Konzentration über die Zeit. Der Indikator ändert beispielsweise seine Farbe von farblos nach grün, wenn er eine gewisse Menge Sauerstoff erfasst hat (z.B. 0,5 Std. 21% O₂ oder 1 Std. 10% O₂).

Selbstverständlich kann in entsprechender Weise eine Charakterisierung eines O₂-Scavenger/Indikatorsystems vorgenommen werden, wobei Scavenger und Indikator gemischt oder getrennt in die Messzelle 6 eingelassen werden können. Zusätzlich zu den schon angegebenen Charakterisierungsgrößen besteht die Möglichkeit, eine Farbänderung des O₂-Indikators in Abhängigkeit zur Restkapazität des O₂-Scavengers zu ermitteln.

Der O₂-Scavenger sorbiert Sauerstoff. Seine absolute Kapazität beträgt 60 cm³/g_{scav}. (siehe Fig. 4). Bei einer erreichten Kapazität von beispielsweise 45 cm³/g_{scav}. ändert der Indikator seine Farbe von farblos nach grün und signalisiert dem Anwender, dass der O₂-Scavenger noch eine Restkapazität von 15 cm³/g_{Scav.} besitzt.

Bei der Charakterisierung von Lebensmittelverpackungen bzw. Getränkeflaschen, die meist aus mehreren polymeren Schichten (Multilayer-Aufbau) bestehen und eine der Schichten das OSI-Material beinhaltet, kann die Verpackung den Platz der Messzelle einnehmen (Verpackung ist ein geschlossenes Kompartiment). Dies bedeutet, dass von der Messzelle der Deckel entfernt wird und die Verpackung mit den Zu- und Abströmöffnungen der Messzelle über Leitungen verbunden wird. In diesem Fall ist also die Verpackung die Messzelle. Diese Charakterisierung ist besonders realistisch, da der von der Umgebung in die Verpackung eintretende Sauerstoff ebenfalls detektiert wird.

Mit der als Ausführungsbeispiel beschriebenen Vorrichtung kann auch der Triggermechanismus von OSI-Materialien bestimmt werden. Dies betrifft ein kombiniertes O₂-Scavenger/Indikator-System (OSI), ein O₂-Scavengersystem (OS) und ein O₂-Indikatorsystem (OI). Bei einem feuchtegetriggerten System wird über die Befeuchtungseinheit sukzessive die relative Feuchte in dem Gasstrom erhöht. Hierdurch wird diejenige relative Feuchte, ab welcher das System aktiviert wird, ermittelt. Bei einem OS- bzw. OSI-System zeigt sich die Aktivierung durch die Abnahme der O2-Konzentration. Bei einem OI- bzw. OSI-System zeigt sich die Aktivierung durch die Farbänderung des Systems. Bei einem UV-getriggerten System wird die Intensität der Strahlung bzw. der Wellenlängenbereich sukzessive erhöht. Die weitere Vorgehensweise ist mit dem feuchtegetriggerten System identisch.

## Patentansprüche

1. Verfahren zur Charakterisierung von OSI-Materialien, bei dem das Material in eine Messzelle (6) eingebracht wird und einem Sauerstoff enthaltenden Gasgemisch ausgesetzt wird und nach einer bestimmten Zeit oder in bestimmten Zeitintervallen ein definierter Volumenteil des Gasgemischs hinsichtlich seiner Sauerstoffkonzentration die zusammen mit der Zeitkomponente eine Charakterisierung für das OSI-Material darstellt in einem Messkreis gemessen wird,
**dadurch gekennzeichnet,**
**dass** das OSI-Material in der Messzelle dem in einem geschlossenen Reaktionskreislauf (1) umgewälzten Gasgemisch ausgesetzt wird und der definierte Volumenteil in einen Gas enthaltenden geschlossenen Messkreislauf (2) zur Messung der Sauerstoffkonzentration überführt wird.

2. Verfahren nach Anspruch 1, **dadurch gekennzeichnet, dass** als OSI-Materialien O₂-Scavenger und/oder O₂-Indikatoren verwendet werden.

3. Verfahren nach Anspruch 1 oder Anspruch 2, **dadurch gekennzeichnet, dass** zur Charakterisierung des Materials in der Messzelle (6), insbesondere eines O₂-Scavengers der Sauerstoffabbau im Gasstrom in Abhängigkeit von der Masse des Materials als Kapazitätsgröße und/oder die zeitliche Änderung des Sauerstoffabbaus als kinetische Größe gemessen wird.

4. Verfahren nach einem der Ansprüche 1 bis 3, **dadurch gekennzeichnet, dass** zur Charakterisierung des Materials in der Messzelle (6), insbesondere eines O₂-Indikators, die Farbe und/oder eine Farbänderung des Materials in Abhängigkeit von der Sauerstoffkonzentration gemessen wird.

5. Verfahren nach Anspruch 4, **dadurch gekennzeichnet, dass** die Farbe und/oder die Farbänderung und/oder die Farbänderung in Abhängigkeit von dem Integral der Sauerstoffkonzentration x Zeit gemessen wird.

6. Verfahren nach einem der Ansprüche 1 bis 5, **dadurch gekennzeichnet, dass** bei O₂-Scavenger/O₂-Indikatorsystemen die Farbänderung des O₂-Indikators in Abhängigkeit zur Restkapazität des O₂-Scavengers bestimmt wird.

7. Verfahren nach einem der Ansprüche 1 bis 6, **dadurch gekennzeichnet, dass** zur Initialisierung des OSI-Materials der Gasstrom in dem Reaktionskreislauf (1) mit Feuchte beaufschlagt wird.

8. Verfahren nach einem der Ansprüche 1 bis 6, **dadurch gekennzeichnet, dass** zur Initialisierung des OSI-Materials dieses in der Messzelle (6) mit UV-Strahlung beaufschlagt wird.

9. Verfahren nach Anspruch 7 oder Anspruch 8, **dadurch gekennzeichnet, dass** der Initialisierungspunkt bzw. -bereich des OSI-Materials abhängig von der relativen Feuchte bzw. der Intensität und/oder des Wellenlängenbereichs der Strahlung bestimmt wird.

10. Vorrichtung zur Charakterisierung von OSI-Materialien mit einem geschlossenen Reaktionskreis und einem geschlossenen Messkreis, wobei der Reaktionskreis (1) eine Vorrichtung (8) zur Zufuhr eines Sauerstoff enthaltenden Gasstroms, eine Pumpe (5) zur Förderung des Gasstroms, und eine Messzelle (6) zur Aufnahme des OSI-Materials aufweist, und der geschlossene Messkreis (2) eine Vorrichtung (9) zur Zufuhr eines Gasstroms, eine Pumpe (10) zur Förderung des Gasstroms und eine Sensoranordnung (11) zur Erfassung von Sauerstoff und eine Auswerteeinheit (12) umfasst, wobei eine Probeschleife (4) mit definiertem Volumen im Reaktionskreis angeordnet ist, die zur Überführung des definierten Volumens des Gasgemischs in dem Reaktionskreis über Ventile (7) in den Messkreis (2) umschaltbar ist.

11. Vorrichtung nach Anspruch 10, **dadurch gekennzeichnet, dass** der Messkreis (2) einen Umschaltzweig (3) aufweist, der über die Ventile (7) in den Reaktionskreis (1) schaltbar ist, wenn der Teil des Reaktionskreises (1) mit definiertem Volumen in den Messkreislauf geschaltet wird.

12. Vorrichtung nach Anspruch 10 oder Anspruch 11, **dadurch gekennzeichnet, dass** die Sensoranordnung (11) mindestens einen sauerstoffsensitiven Sensor und die Auswerteeinheit (12) einen Integrator enthält.

13. Vorrichtung nach einem der Ansprüche 10 bis 12, **dadurch gekennzeichnet, dass** die Vorrichtung (8) zur Zufuhr des Sauerstoff enthaltenden Gasstroms in den Reaktionskreis (1) mit einer Befeuchtungseinheit (15) verbunden ist, die den Gasstrom mit einer für die Initialisierung des Materials in der Messzelle (6) notwendigen Befeuchtung beaufschlagt.

14. Vorrichtung nach einem der Ansprüche 10 bis 13, **dadurch gekennzeichnet, dass** die Messzelle (6) für vorgebbare Wellenlängenbereiche durchlässig ist.

15. Vorrichtung nach Anspruch 14, **dadurch gekennzeichnet, dass** der Messzelle (6) eine UV-Strahlungsquelle zugeordnet ist, die das Material zu dessen Initialisierung bestrahlt.

16. Vorrichtung nach einem der Ansprüche 10 bis 15, **dadurch gekennzeichnet, dass** der Messzelle eine Vorrichtung zur Messung der Farbe und/oder der Farbänderung des Materials zugeordnet ist.

17. Vorrichtung nach einem der Ansprüche 10 bis 16, **dadurch gekennzeichnet, dass** die Bauteile des Reaktionskreises (1) und des Messkreises (2) gekapselt sind.

## Claims

1. Method for characterising OSI materials, in which the material is placed in a measuring cell (6) and is exposed to a gas mixture containing oxygen, and after a specific time or in specific time intervals a defined part by volume of the gas mixture is measured in a measuring circuit with respect to its oxygen concentration, which together with the time component represents a characterisation of the OSI material, **characterised in that** in the measuring cell the OSI material is exposed to the gas mixture circulated in a closed reaction circuit (1) and the defined part by volume is moved into a closed measuring circuit (2) containing gas for measurement of the oxygen concentration.

2. Method according to claim 1, **characterised in that** O₂ scavengers and/or O₂ indicators are used as OSI materials.

3. Method according to claim 1 or claim 2, **characterised in that** for characterisation of the material in the measuring cell (6), in particular an O₂ scavenger, the breakdown of oxygen in the gas flow is measured as capacity value and/or the time change of the oxygen breakdown is measured as kinetic value, as a function of the mass of the material.

4. Method according to one of claims 1 to 3, **characterised in that** for characterisation of the material in the measuring cell (6), in particular an O₂ indicator, the colour and/or a colour change of the material is measured as a function of the oxygen concentration.

5. Method according to claim 4, **characterised in that** the colour and/or the colour change and/or the colour change? is measured as a function of the integral of the oxygen concentration x time.

6. Method according to one of claims 1 to 5, **characterised in that** in the case of O₂ scavenger/O₂ indicator systems, the colour change of the O₂ indicator is determined as a function of the residual capacity of the O₂ scavenger.

7. Method according to one of claims 1 to 6, **characterised in that** for initialisation of the OSI material, the gas flow in the reaction circuit (1) is subjected to moisture.

8. Method according to one of claims 1 to 6, **characterised in that** for initialisation of the OSI material, this is subjected to UV radiation in the measuring cell (6).

9. Method according to claim 7 or claim 8, **characterised in that** the initialisation point or range of the OSI material is determined in dependence on the relative humidity or the intensity and/or the wavelength range of the radiation.

10. Device for characterising OSI materials with a closed reaction circuit and a closed measuring circuit, wherein the reaction circuit (1) has a device (8) for supplying a gas flow containing oxygen, a pump (5) for transporting the gas flow and a measuring cell (6) for receiving the OSI material, and the closed measuring circuit (2) comprises a device (9) for supplying a gas flow, a pump (10) for transporting the gas flow, and a sensor arrangement (11) for detecting oxygen and an evaluation unit (12), wherein a sample loop (4) with a defined volume is arranged in the reaction circuit and can be switched over to transfer the defined volume of the gas flow in the reaction circuit via valves (7) into the measuring circuit (2).

11. Device according to claim 10, **characterised in that** the measuring circuit (2) has a switchover branch (3), which is switchable via the valves (7) into the reaction circuit (1) when the part of the reaction circuit (1) with defined volume is switched into the measuring circuit.

12. Device according to claim 10 or claim 11, **characterised in that** the sensor arrangement (11) contains at least one oxygen-sensitive sensor and the evaluation unit (12) contains an integrator.

13. Device according to one of claims 10 to 12, **characterised in that** to supply the gas flow containing oxygen into the reaction circuit (1) the device (8) is connected to a humidifier unit (15), which subjects the gas flow to a humidity necessary for the initialisation of the material in the measuring cell (6).

14. Device according to one of claims 10 to 13, **characterised in that** the measuring cell (6) is transparent to predetermined wavelength ranges.

15. Device according to claim 14, **characterised in that** the measuring cell (6) has an associated UV radiation source, which irradiates the material for its initialisation.

16. Device according to one of claims 10 to 15, **characterised in that** the measuring cell has an associated device for measuring the colour and/or the colour change of the material.

17. Device according to one of claims 10 to 16, **characterised in that** the components of the reaction circuit (1) and the measuring circuit (6) are encapsulated.

## Revendications

1. Procédé pour caractériser les matériaux OSI selon lequel on introduit le matériau dans une cellule de mesure (6) et on l'expose à un mélange gazeux contenant de l'oxygène et, après un certain temps ou certains intervalles de temps, on mesure une partie volumique définie du mélange gazeux pour déterminer sa concentration en oxygène représentant avec la composante de temps, une caractéristique du matériau OSI, en mesurant dans un circuit de mesure,
**caractérisé en ce que**
on expose le matériau OSI dans la cellule de mesure au mélange gazeux circulant dans un circuit fermé de réaction (1), et on transfère la partie volumique définie dans un circuit de mesure (2), fermé contenant un gaz pour mesurer la concentration en oxygène.

2. Procédé selon la revendication 1,
**caractérisé en ce que**
comme matériau OSI on utilise des matériaux épuisant l'oxygène O₂ et/ou des indicateurs d'oxygène O₂.

3. Procédé selon la revendication 1 ou selon la revendication 2,
**caractérisé en ce que**
pour caractériser le matériau dans la cellule de mesure (6), en particulier d'un matériau épuisant l'oxygène O₂ on mesure la disparition de l'oxygène dans la veine de gaz, en fonction de la masse du matériau, comme grandeur de capacité et/ou la variation dans le temps de la décomposition de l'oxygène comme grandeur cinématique.

4. Procédé selon l'une des revendications 1 à 3,
**caractérisé en ce que**
pour caractériser le matériau dans la cellule de mesure (6) notamment d'un indicateur d'oxygène O₂, on mesure la teinte et/ou une variation de teinte du matériau en fonction de la concentration en oxygène.

5. Procédé selon la revendication 4,
**caractérisé en ce qu'**
on mesure la teinte et/ou la variation de teinte et/ou la variation de teinte en fonction de l'intégrale de la concentration en oxygène multipliée par le temps.

6. Procédé selon l'une des revendications 1 à 5,
**caractérisé en ce que**
pour des systèmes d'agent épuisant l'oxygène O₂/indicateur d'oxygène O₂, on détermine la variation de teinte de l'indicateur O₂ en fonction de la capacité résiduelle de l'agent épuisant l'oxygène O₂.

7. Procédé selon l'une des revendications 1 à 6,
**caractérisé en ce que**
pour initialiser le matériau OSI, on humidifie la veine de gaz dans le circuit de réaction (1).

8. Procédé selon l'une des revendications 1 à 6,
**caractérisé en ce que**
pour initialiser le matériau OSI, on lui applique un rayonnement ultraviolet UV dans la cellule de mesure (6).

9. Procédé selon la revendication 7 ou la revendication 8,
**caractérisé en ce que**
l'on détermine le point d'initialisation ou la zone d'initialisation du matériau OSI en fonction de l'humidité relative, de l'intensité et/ou de la plage des longueurs d'ondes du rayonnement.

10. Procédé pour caractériser les matériaux OSI, comprenant un circuit de réaction fermé et un circuit de mesure fermé,
le circuit de réaction (1) comportant un dispositif (8) pour fournir une veine de gaz contenant de l'oxygène, une pompe (5) pour transférer la veine de gaz et une cellule de mesure (6) pour recevoir le matériau OSI et
le circuit de mesure fermé (2) comporte un dispositif (9) pour fournir une veine de gaz, une pompe (10) pour transférer la veine de gaz et un dispositif de capteur (11) pour saisir l'oxygène et une unité d'exploitation (12),
une boucle d'échantillonnage (4) de volume défini étant prévue dans le circuit de réaction pour transférer le volume défini du mélange gazeux dans le circuit de réaction par commutation par des vannes (7) dans le circuit de mesure (2).

11. Dispositif selon la revendication 10,
**caractérisé en ce que**
le circuit de mesure (2) comporte une branche de commutation (3) commutée par les soupapes (7) dans le circuit de réaction (1) lorsque la partie du circuit de réaction (1) est branchée avec un volume défini dans le circuit de mesure.

12. Dispositif selon la revendication 10 ou la revendication 11,
**caractérisé en ce que**
les dispositifs de capteur (11) comportent au moins un capteur sensible à l'oxygène et l'unité d'exploitation (12) comporte un intégrateur.

13. Dispositif selon l'une des revendications 10 à 12,
**caractérisé en ce que**
le dispositif (8) est relié à une unité d'humidification (15) pour fournir la veine de gaz contenant l'oxygène au circuit de réaction (1), l'unité d'humidification appliquant à la veine de gaz l'humidité nécessaire pour l'initialisation du matériau dans la cellule de mesure (6).

14. Dispositif selon l'une des revendications 10 à 13,
**caractérisé en ce que**
la cellule de mesure (6) est transparente pour des plages de longueur d'ondes prédéfinies.

15. Dispositif selon la revendication 14,
**caractérisé en ce que**
la cellule de mesure (6) est combinée à une source de rayonnement ultraviolet UV qui irradie le matériau pour l'initialiser.

16. Dispositif selon l'une des revendications 10 à 15,
**caractérisé par**
un dispositif de mesure de la teinte et/ou de la variation de teinte du matériau associé à la cellule de mesure.

17. Dispositif selon l'une des revendications 10 à 16,
**caractérisé en ce que**
les composants du circuit de réaction (1) et du circuit de mesure (2) sont encapsulés.
